(19) Europäisches Patentamt
European Patent Office
Office européen des brevets

(11) Publication number: **0 108 847**
**B1**

(12) # EUROPEAN PATENT SPECIFICATION

(45) Date of publication of patent specification: **18.06.86**

(21) Application number: **82306080.1**

(22) Date of filing: **15.11.82**

(51) Int. Cl.[4]: **C 07 C 51/10**, C 07 C 53/08, C 07 C 53/122, C 07 C 67/36, C 07 C 69/14, C 07 C 69/24, B 01 J 23/46

(54) **Production of acetic acid, propionic acid and their esters.**

(43) Date of publication of application:
**23.05.84 Bulletin 84/21**

(45) Publication of the grant of the patent:
**18.06.86 Bulletin 86/25**

(84) Designated Contracting States:
**BE DE FR GB IT NL**

(56) References cited:
**GB-A-2 029 409**
**US-A-4 136 104**

**CHEMICAL ABSTRACTS, vol. 95, no. 15, 12th October 1981, page 616, no. 132245e, Columbus, Ohio, USA J.F. KNIFTON: "Syngas reactions. III. Ruthenium catalyzed homologation of aliphatic carboxylic acids"**

(73) Proprietor: **TEXACO DEVELOPMENT CORPORATION**
**2000 Westchester Avenue**
**White Plains New York 10650 (US)**

(72) Inventor: **Knifton, John Frederick**
**10900 Catskill Trail**
**Austin Texas 78750 (US)**
Inventor: **Lin, Jiang-Jen**
**2617 Oak Meadow Dr.**
**Round Rock Texas 78664 (US)**

(74) Representative: **Brock, Peter William et al**
**Michael Burnside & Partners 2 Serjeants' Inn**
**Fleet Street**
**London EC4Y 1HL (GB)**

EP 0 108 847 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention concerns an improved process for preparing carboxylic acids, especially acetic and propionic acids, and their esters by a single stage reaction of oxides of carbon with hydrogen in presence of a catalyst system.

There are ever-increasing efforts to provide new methods of making carboxylic acids, such as acetic acid and esters thereof, which are particularly useful in preparing a wide variety of organic compounds, such as cellulose acetate, and vinyl acetate. An ever-present aim is to prepare such material in relatively high yields directly from carbon monoxide and hydrogen utilizing a catalyst system providing good selectivity.

A number of processes have been described in the literature for manufacturing carboxylic acids and esters from carbon monoxide and alcohols or from carbon monoxide and hydrogen. For example, in U.S. Application 3,717,670, a method for preparing such carboxylic acids is disclosed in which an alcohol and carbon monoxide are reacted in the presence of a catalyst composition consisting of a rhodium compound and, for example, chromium trioxide. According to U.S. Application 4,096,164, a reaction product containing acetic acid, acetaldehyde and/or ethanol is formed when hydrogen and carbon monoxide are passed over a catalyst comprising rhodium in combination with molybdenum and/or tungsten. A similar method is described in U.S. Application 4,014,913 in which a rhodium-manganese catalyst is employed. The reaction of carbon monoxide and hydrogen in the presence of rhodium metal catalyst to give a liquid product containing a substantial proportion of acetic acid, ethanol and/or acetaldehyde is disclosed in U.S. Application 4,246,186. Likewise in U.S. Application 4,162,262 it is noted that the reaction of hydrogen and carbon monoxide in the presence of a catalyst comprising thorium and/or uranium yields a product containing a large amount of product with two carbon atoms. Other processes for preparing carboxylic acids from carbon monoxide and hydrogen are disclosed in US—A—4101450 and in NL—A—7500918.

One serious problem associated with synthesis gas operations in the past has been the non-selectivity of the product distribution, since high activity catalysts generally yield a liquid product containing numerous different compounds. Thus, complicated recovery schemes are necessary to separate the desired products and the overall yield of the valuable organic products is low.

Within recent years it has been shown that catalysts comprising ruthenium compounds dispersed in low melting quaternary ammonium or phosphonium bases or salts can be used for the selective conversion of carbon monoxide and hydrogen (syngas) into alkanols or glycols, or the homologation of alkanols. Reference may be made for example to the following published documents: EP—A—0022038: homologation of methanol in the presence of a catalyst comprising ruthenium and cobalt compounds, an alkyl halide and an ionic (alkali metal, alkaline earth metal or quaternary) halide;

EP—A—0055514: formation of glycols in the presence of a catalyst comprising ruthenium or rhodium acetylacetonate dispersed in an ammonium or phosphonium salt;

EP—A—0056679: homologation of methanol in the presence of a catalyst comprising a ruthenium compound and a cobalt halide dispersed in a quaternary ammonium or phosphonium base or salt.

More recently, we have shown in our pending British Applications 8213656; 8213657 and 8213658 (unpublished at the filing date of this Application), that alkanols can be produced from synthesis gas in the presence of a ruthenium compound and a co-catalyst, dispersed in a quaternary ammonium or phosphonium base or salt. The co-catalyst may be a halogen-free cobalt compound (GB 8213656), a halogen-free titanium or zirconium compound (GB 8213657), or a halogen-free rhenium or manganese compound (GB 8213658).

It has now surprisingly been found that if a halogen-containing co-catalyst is employed, or if a halogen-free co-catalyst is employed in association with iodine or an iodine-containing compound, syngas can be selectively converted into carboxylic acids (especially acetic and propionic acids) and esters thereof, rather than alcohols.

The present invention therefore provides a process for reacting mixtures of carbon monoxide and hydrogen, at a temperature of at least 150°C and a pressure of at least 35 bars in the presence of a catalyst comprising a ruthenium compound and a co-catalyst dispersed in a low melting quaternary ammonium or phosphonium base or salt, characterized in that the co-catalyst is a halogen-containing cobalt, titanium or zirconium compound or a halogen-containing or halogen-free cobalt titanium or zirconium compound being employed with iodine or an iodine-containing compound and the mixtures of carbon monoxide and hydrogen do not include other gases that are reactive under co-hydrogenation conditions, whereby said carbon monoxide and hydrogen are selectively converted into acetic acid, propionic acid and esters thereof.

Catalysts that are suitable in the practice of this invention contain (a) ruthenium and (b) cobalt, titanium or zirconium. The catalysts may be chosen from a wide variety of organic or inorganic compounds, complexes, etc., as will be shown and illustrated below. It is only necessary that the catalyst precursor actually employed contains said metals in any of their ionic states. The actual catalytically active species is then believed to comprise ruthenium and cobalt, titanium or zirconium in complex combination with carbon monoxide and hydrogen. The most effective catalysis is believed to be achieved where ruthenium and titanium or zirconium hydrocarbonyl species are solubilized in a quaternary salt under reaction conditions.

The ruthenium catalyst precursors may take many different forms. For instance, the ruthenium may be added to the reaction mixture in an oxide form, as in the case of, for example, ruthenium(IV) oxide hydrate, anhydrous ruthenium(IV) dioxide and ruthenium(VIII) tetraoxide. Alternatively, it may be added as the salt of a mineral acid, as in the case of ruthenium(III) chloride hydrate, ruthenium(III) bromide, ruthenium(III) triiodide, tricarbonyl ruthenium(II) iodide, anhydrous ruthenium(III) chloride and ruthenium nitrate, or as the salt of a suitable organic carboxylic acid, for example, ruthenium(III) acetate, ruthenium naphthenate, ruthenium valerate and ruthenium complexes with carbonyl-containing ligands, such as ruthenium(III) acetylacetonate. The ruthenium may also be added to the reaction zone as a carbonyl or hydrocarbonyl derivative. Here, suitable examples include triruthenium dodecacarbonyl and other hydrocarbonyls such as $H_2Ru_4(CO)_{13}$ and $H_4Ru_4(CO)_{12}$, and substituted carbonyl species such as the tricarbonylruthenium(II) chloride dimer, $[Ru(CO)_3Cl_2]_2$.

Preferred ruthenium-containing compounds include oxides of ruthenium, ruthenium salts of an organic carboxylic acid and ruthenium carbonyl or hydrocarbonyl derivatives. Among these, particularly preferred are ruthenium(IV) dioxide hydrate, ruthenium(VIII) tetraoxide, anhydrous ruthenium(IV) oxide, ruthenium acetate, ruthenium(III) acetylacetonate, and triruthenium dodecacarbonyl.

Cobalt halides useful in the catalyst systems of this invention include cobalt(II) iodide, cobalt(II) bromide, cobalt(II) bromide hydrate, cobalt(II) chloride hydrate, anhydrous cobalt(II) chloride as well as mixtures thereof.

Alternatively, in place of the cobalt halide, it is possible to employ a halogen-free cobalt compound plus, as a third component, iodine or an iodine compound. Suitable halogen-free cobalt compounds include cobalt oxides, for example, cobalt(II) oxide or cobalt(II, III) oxide; cobalt salts of mineral acids such as cobalt nitrate hydrate, and cobalt sulphate; cobalt salts of organic carboxylic acids, for example, cobalt(II) formate, cobalt(II) acetate, cobalt(II) oxalate, and cobalt naphthenate, as well as cobalt complexes with carbonyl-containing ligands as in the case of cobalt(III) acetylacetonate. The cobalt may also be added as cobalt carbide, cobalt(II) carbonate or as a carbonyl or hydrocarbonyl derivative. Here suitable examples include dicobalt octacarbonyl, cobalt hydrocarbonyl and substituted cobalt carbonyls.

Elemental iodine can be employed in this embodiment. Suitable iodine-containing compounds include organic iodides such as alkyl iodides, acyl iodides and aryl iodides. Specific examples of suitable organic iodides are such alkyl iodides as methyl iodide, and ethyl iodide.

Optionally in the first-described embodiment of the process of this invention iodine or an iodine compound of the kind illustrated above, can be added to the catalyst combination previously outlined consisting of a ruthenium compound taken from the class described above and a halogen-containing cobalt compound also of the type previously described. The advantage of adding this third, iodine or iodide, component to the catalyst combination, even when a cobalt halide is being used as co-catalyst, is the improved selectivity to acetic acid that may be realized.

The halogen-containing titanium and zirconium salt catalyst precursors may take many different forms. For instance, suitable halogen-containing titanium or zirconium salts include titanium and zirconium halides such as titanium dichloride oxide, zirconium dichloride oxide, $(ZrOCl_2 \cdot 4H_2O)$; zirconium(IV) bromide, zirconium(IV) chloride, titanium(IV) bromide, titanium(III) chloride and titanium(IV) chloride. Alternatively, the titanium and zirconium halide salts may be in complexed form with other coordinating ligands. Suitable halogen-containing titanium or zirconium complexes include bis(cyclopentadienyl) zirconium dichloride, bis(cyclopentadienyl)zirconium hydrochloride, bis(indenyl)titanium dichloride, cyclopentadienyltitanium trichloride and titanocene dichloride.

Preferred titanium and zirconium halogen-containing salts include bis(cyclopentadienyl)zirconium dichloride, bis(cyclopentadienyl)titanium dichloride, bis(cyclopentadienyl)zirconium hydrochloride and bis(cyclopentadienyl)titanium hydrochloride.

If desired, mixtures of the ruthenium-containing compounds as well as the mixtures of the halogen-containing titanium and/or zirconium salts may be employed.

Preferably, the composition of the catalyst system comprising a ruthenium-containing compound and a halogen-containing titanium or zirconium salt is such that the atomic ratio of total halogen, as derived from the ruthenium-containing compound and the halogen-containing titanium or zirconium salt to the total of Ru+Ti or Zr should not exceed 2:1.

Alternatively, the co-catalyst can be a halogen-free titanium or zirconium compound plus, as a third component, iodine or an iodine compound. Suitable halogen-free titanium or zirconium compounds may include zirconium diacetate oxide, zirconium dinitrate oxide, zirconium(IV) 2,4-pentanedionate, titanium(IV) 2,4-pentanedionate zirconium(IV) i-propoxide, zirconium(IV) n-propoxide, titanium(IV) butoxide, titanium(IV) cresylate, titanium(IV) ethoxide, titanium(IV) methoxide, zirconium(IV) methoxide, titanium(IV) n-nonylate, titanium oxalate, titanium(IV) stearylate and titanium oxide. Suitable iodine sources have been mentioned above in connection with the cobalt-containing co-catalyst.

Generally, in the practice of this invention, the ruthenium-containing compound and either the halogen-containing cobalt, titanium or zirconium salt, or the halogen-free cobalt, titanium or zirconium compound plus source of iodine, are first dispersed in a low-melting quaternary phosphonium or ammonium base or salt before their catalytic use in making carboxylic acids. It is interesting to note that the halogen-containing cobalt, titanium, or zirconium salt alone when dispersed in the low-melting salt or

base, has little if any activity in promoting the manufacture of acetic or propionic acids or their esters from synthesis gas.

The quaternary phosphonium or ammonium base or salt must be relatively low melting, that is, melt at a temperature less than about the temperature of reaction of making aliphatic carboxylic acids. Usually the quaternary compound has a melting point less than 180°C and most often has a melting point less than 150°C.

Suitable quaternary phosphonium salts have the formula:

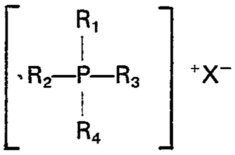

where $R_1$, $R_2$, $R_3$ and $R_4$ are the same or different organic radicals, particularly alkyl, aryl or alkaryl radicals bonded to the phosphorus atom, and X is an anion. The organic radicals useful in this instance include branched or linear alkyl radicals having 1 to 20 carbon atoms e.g. methyl, ethyl, *n*-butyl, *iso*-butyl, octyl, 2-ethylhexyl and dodecyl. Tetraethylphosphonium bromide and tetrabutylphosphonium bromide are typical commercially-available examples. The corresponding quaternary phosphonium acetates, hydroxides, nitrates, chromates, tetrafluoroborates and other halides, such as the corresponding chlorides, and iodides, are also satisfactory in this instance. Also useful are the quaternary ammonium bases and salts corresponding to the above phosphonium compounds.

Equally useful are the phosphonium and ammonium salts containing phosphorus or nitrogen bonded to a mixture of alkyl, aryl and alkaryl radicals. Said aryl and alkaryl radicals may each contain 6 to 20 carbon atoms. The aryl radicals may each contain 6 to 20 carbon atoms. The aryl radical is most commonly phenyl. The alkaryl group may comprise phenyl substituted with one or more $C_1$—$C_{10}$ alkyl substituents, bonded to the phosphorus or nitrogen atom through the aryl function.

Illustrative examples of suitable quaternary phosphonium and ammonium bases and salts include tetrabutylphosphonium bromide, heptyltriphenylphosphonium bromide, tetrabutylphosphonium iodide, tetrabutylphosphonium chloride, tetrabutylphosphonium nitrate, tetrabutylphosphonium hydroxide, tetrabutylphosphonium chromate, tetrabutylphosphonium tetrafluoroborate, tetrabutylphosphonium acetate, tetrabutylammonium bromide and tetramethylammonium hydroxide, pentahydrate and trimethyldodecylammonium bromide.

The preferred quaternary salts are generally tetraalkylphosphonium salts containing alkyl groups having 1—6 carbon atoms, such as methyl, ethyl, and butyl. Tetrabutylphosphonium salts, such as tetrabutylphosphonium bromide, are most preferred for the practice of this invention. Preferred, tetrabutylphosphonium salts or bases include the bromide, chloride, iodide, acetate and chromate salts and hydroxide base.

Generally, in this catalyst system the molar ratio of the ruthenium compound to the quaternary phosphonium or ammonium salt or base will be from 1:0.01 to 1:100, or even higher, and, preferably, will be from 1:0.5 to 1:20.

The quantity of ruthenium compound employed in the instant invention is not critical and may vary over a wide range. In general, the novel process is desirably conducted in the presence of a catalytically effective quantity of the active ruthenium species and of the cobalt, titanium, or zirconium which gives the desired product in reasonable yield, provided the atomic ratio of the total halogen as derived from ruthenium-containing compound and the halogen-containing cobalt, titanium or zirconium salt to the total of Ru+Co, Ti or Zr does not exceed 2:1. The reaction proceeds when employing as little as $1\times10^{-6}$ weight percent, and even lesser amounts, of ruthenium together with $1\times10^{-6}$ weight percent or less of cobalt, titanium, or zirconium, basis the total weight of the reaction mixture. The upper concentration is dictated by a variety of factors including catalyst cost, partial pressures of carbon monoxide and hydrogen, operating temperature, etc. A ruthenium concentration of from $1\times10^{-5}$ to 5 weight percent in conjunction with a cobalt, titanium or zirconium concentration of from $1\times10^{-5}$ to 5 weight percent, based on the total weight of reaction mixture, is generally desirable in the practice of this invention. The preferred ruthenium-to-cobalt, titanium or zirconium atomic ratio is from 10:1 to 1:10.

In those cases where the catalytic reaction mixture contains iodine or an iodine compound, the synthesis of acetic and propionic acids from $CO/H_2$ may best be realized when the atomic ratio of total iodide in the system, as derived from the ruthenium-containing compound, the cobalt titanium or zirconium compound, the low-melting quaternary phosphonium or ammonium salt and the optionally added iodine or iodine compound, to the total cobalt titanium or zirconium content does not exceed 6:1.

The temperature range which can usefully be employed in these syntheses is a variable dependent upon other experimental factors, including the pressure, and the concentration and choice of the particular species of the ruthenium catalyst as well as the particular co-catalyst. The range of operability is from 150 to 350°C when superatmospheric pressure of syngas are employed. A narrow range of 180 to 250°C represents the preferred temperature range.

Superatmospheric pressures of at least 35 bars or greater lead to substantial yields of acetic and

propionic acids and their esters by the process of this invention. A preferred operating range is from 135 to 700 bars, although higher pressures also provide useful yields of the desired acids and esters. With titanium and zirconium compounds, the preferred maximum pressure is slightly lower at 625 bars.

The relative amounts of carbon monoxide and hydrogen which may be initially present in the syngas mixture are variable, and these amounts may be varied over a wide range. In general, the mole ratio of $CO:H_2$ is in the range from 20:1 to 1:20, preferably from 5:1 to 1:5, although ratios outside these ranges may also be employed. Particularly in continuous operations, but also in batch experiments, the carbon monoxide-hydrogen gaseous mixtures may also be used in conjunction with up to 50 percent by volume of one or more other gases. These other gases may include one or more inert gases such as nitrogen, argon, or neon, or they may include gases that may not undergo reaction under CO hydrogenation conditions.

Esters of acetic acid and propionic acid formed during the course of this process include methyl acetate, ethyl acetate, propyl acetate, methyl propionate, ethyl propionate and propyl propionate, etc. These esters and the individual acids formed can be conveniently recovered from the reaction mixture by distillation or extraction.

The novel process of this invention can be conducted in a batch, semi-continuous or continuous fashion. The catalyst may be initially introduced into the reaction zone batchwise, or it may be continuously or intermittently introduced into such a zone during the course of the synthesis reaction. Operating conditions can be adjusted to optimize the formation of the desired acids and esters and said materials may be recovered, as previously pointed out, by methods well known in the art, such as distillation, fractionation, and extraction. A fraction rich in the catalyst components may then be recycled to the reaction zone, if desired, and additional products generated.

The products have been identified in this work by one or more of the following analytical procedures, viz. gas-liquid phase chromatograph (glc), infrared (ir), mass spectrometry, nuclear magnetic resonance (nmr) and elemental analyses, or a combination of these techniques. Analyses have, for the most part, been by parts in weight; all temperatures are in degrees centigrade and all pressures in bars.

The following Examples illustrate various embodiments of this invention.

Example 1

This Example illustrates the synthesis of acetic acid in high yield together with propionic acid and their esters directly from synthesis gas using a zirconium-ruthenium containing catalyst dispersed in tetrabutylphosphonium bromide (m.p. 100°C).

A mixture of ruthenium(IV) oxide (4 mmoles) and bis(cyclopentadienyl)zirconium hydrochloride (4 mmoles) dispersed in tetrabutylphosphonium bromide (10.0 g) was charged to a glass liner, under nitrogen purge, and transferred to a 450 ml capacity pressure reactor equipped with heating and means of agitation. The reactor was sealed, flushed with $CO/H_2$ mixture and pressurized to 277 bars with a 1:1 molar $CO/H_2$ mixture. The mixture was heated to 220°C with rocking, the pressure allowed to rise to 458.5 bars, and the reactor held at temperature for 18 hours.

On cooling, the reactor pressure (168 bars) was noted, a typical gas sample taken and the excess gas removed. A dark-green liquid product (15.8 g) was recovered and samples were analyzed by glc and Karl Fischer titration. The following results were obtained:

45.3 wt.% acetic acid
3.2 wt.% propionic acid
9.6 wt.% ethyl acetate
5.6 wt.% ethyl propionate
3.3 wt.% propyl propionate
1.9 wt.% water

A small quantity of lighter, water-white, liquid phase was identified as primarily hydrocarbon.

Analysis of typical off-gas samples showed the presence of:

39% hydrogen
23% carbon monoxide
26% carbon dioxide
7% methane

Since the total catalyst charge to the glass liner was 11.8 g, the yield of liquid products was calculated to be:

$$\frac{15.8-11.8}{11.8}\times 100=34\%.$$

Example 2

This Example illustrates a second synthesis of acetic acid in high yield together with propionic acid and their esters directly from synthesis gas using a zirconium-ruthenium containing catalyst dispersed in tetrabutylphosphonium bromide.

A mixture of ruthenium(IV) oxide (4 mmoles) and bis(cyclopentadienyl)zirconium dichloride (4 mmoles) dispersed in tetrabutylphosphonium bromide (10.0 g) was charged to a glass liner, under nitrogen

purge, and transferred to a 450 ml capacity pressure reactor equipped with heating and means of agitation. The reactor was sealed, flushed with $CO/H_2$ mixture and pressurized to 277 bars, with a 1:1 molar $CO/H_2$ mixture. The mixture was heated to 220°C with rocking, the pressure allowed to rise to 489 bars and the reactor held at temperature for 18 hours.

On cooling, the reactor pressure (189 bars) was noted, a typical gas sample taken and the excess gas removed. A dark-green liquid product (17.1 g) was recovered and samples were analyzed by glc and Karl Fischer titration. The following results were obtained:

48.7 wt.% acetic acid
8.9 wt.% propionic acid
9.2 wt.% ethyl acetate
1.1 wt.% ethyl propionate
2.7 wt.% propyl propionate
1.6 wt.% water

A small quantity of lighter, water-white, liquid phase was identified as primarily hydrocarbon.

Analysis of typical off-gas samples showed the presence of:

46% hydrogen
42% carbon monoxide
10% carbon dioxide
1.8% methane

Since the total catalyst charge to the glass liner was 11.9 g, the yield of liquid products was calculated to be: 44%

Fractional distillation of a 7.3 g sample of the crude liquid product, under 0.1 mm Hg vacuum, produced a distillate sample comprising >80% purity acetic acid.

Examples 3—5

Following the general procedure of Example 1, three additional Examples were run employing ruthenium(IV) oxide, ruthenium(III) acetylacetonate and triruthenium dodecacarbonyl coupled with the halide-containing titanium and zirconium salts, bis(cyclopentadienyl)titanium dichloride and bis(cyclopentadienyl)zirconium dichloride. The results obtained are set out in Table 1, below. Acetic acid is a major product of CO hydrogenation in all three cases.

TABLE 1

| Example[a] | Catalyst | Melt | Liquid product composition (weight %)[b] HOAc | PrOOH | MeOAc | EtOAc/ MeOOPr | PrOAc/ EtOOPr | BuOOH/ PrOOPr | $H_2O$ | Liquid yield (%) |
|---|---|---|---|---|---|---|---|---|---|---|
| 1 | $RuO_2$—$Cp_2ZrHCl$ | $Bu_4PBr$ | 45.3 | 3.2 | 0.8 | 9.6 | 5.6 | 3.3 | 1.9 | 34 |
| 2 | $RuO_2$—$Cp_2ZrCl_2$ | $Bu_4PBr$ | 48.7 | 8.9 | — | 9.2 | 1.1 | 2.7 | 1.6 | 44 |
| 3 | $RuO_2$—$Cp_2TiCl_2$ | $Bu_4PBr$ | 13.4 | 0.5 | 3.0 | 31.8 | 13.9 | 4.1 | 1.2 | 55 |
| 4 | $Ru(acac)_3$—$Cp_2ZrCl_2$ | $Bu_4PBr$ | 24.7 | 8.9 | 1.4 | 26.2 | 7.7 | 4.7 | 1.1 | 37 |
| 5 | $Ru_3(CO)_{12}$—$Cp_2ZrCl_2$ | $Bu_4PBr$ | 17.1 | 1.2 | 5.7 | 33.8 | 11.4 | 4.5 | 5.5 | 44 |
| 6 | $Cp_2ZrHCl$ | $Bu_4PBr$ | — | — | — | — | — | — | — | <5 |
| 7 | $Cp_2TiCl_2$ | $Bu_4PBr$ | — | — | — | — | — | — | — | <5 |

[a]Charge: Ruthenium, 4.0 mmoles; zirconium/titanium, 4.0 mmoles; tetrabutylphosphonium bromide, 10 g. Run Conditions: 277 bars, 1:1 molar ($CO/H_2$), initial pressure; 220°C, 18 hours.
[b]Designations: Acetic Acid (HOAc); Propionic Acid (PrOOH); Butyric Acid (BuOOH); Acetate Esters (MeOAc, EtOAc, PrOAc); Propionate Esters (MeOOPr, EtOOPr, PrOOPr).
Cp=cyclopentadienyl.
acac=acetylacetonyl.

Comparative Examples 6 and 7

In these Comparative Examples, the catalyst utilized consisted only of the halide-containing titanium or zirconium compound dispersed in the low-melting quaternary. There was no ruthenium catalyst component. No liquid product was formed in the absence of the ruthenium component.

In a typical Example (6), a mixture of bis(cyclopentadienyl)zirconium hydrochloride (4 mmoles) dispersed in tetrabutylphosphonium bromide (10 g) was charged to a glass liner, under $N_2$ purge, and transferred to a 450 ml capacity pressure reactor. The reactor was sealed, flushed with a $CO/H_2$ mixture, pressurized to 277 bars with 1:1 molar $CO/H_2$ mixture and heated to 220°C with rocking for 18 hours.

On cooling, the reactor pressure (277 bars) was noted, a typical gas sample taken, and the excess gas removed. The product within the glass liner consisted of 11.2 g of a brown solid. There was no liquid product in this case.

Pertinent data relating to these Examples are shown in Table 1.

Example 8

A mixture of ruthenium(IV) oxide (4 mmoles) and zirconium(IV) bromide (4 mmoles) dispersed in tetrabutylphosphonium bromide (10.0 g) was charged to a glass liner, under nitrogen purge, and transferred to a 450 ml capacity pressure reactor equipped with heating and means of agitation. The reactor was sealed, flushed with $CO/H_2$ mixture and pressurized to 277 bars with a 1:1 molar $CO/H_2$ mixture. The mixture was heated to 220°C with rocking, the pressure allowed to rise to 408.5 bars, and the reactor held at temperature for 18 hours.

On cooling the reactor pressure (97.5 bars) was noted and the excess gas removed. The grey liquid product suspension (31.5 g) was recovered and samples analyzed by glc and Karl Fischer titration. Both acetic and propionic acids were identified as being present in the liquid product fraction.

Example 9

A mixture of ruthenium chloride, hydrate (4.0 mmoles), bis(cyclopentadienyl)zirconium dichloride (4.0 mmoles), and tetrabutylphosphonium bromide (10 g), were charged to a glass liner, under $N_2$ purge, and transferred to the same 450 ml capacity pressure reactor as in Example 1. The reactor was sealed, flushed with $CO/H_2$ mixture, pressurized to 277 bars with 1:1 molar $CO/H_2$ mixture and heated to 220°C with rocking for 18 hours.

On cooling, the reactor pressure (71 bars) was noted and the excess gas removed. The dark-green liquid product suspension (32.6 g) was recovered and samples analyzed by glc and Karl Fischer titration. Acetic acid was identified as being present in this product liquid.

Example 10

A mixture of ruthenium(IV) oxide (4 mmoles) and zirconium dichloride, oxide ($ZrCl_2O \cdot 4H_2O$, 4 mmoles) dispersed in tetrabutylphosphonium bromide (10.0 g) was charged to a glass liner, under nitrogen purge, and transferred to a 450 ml capacity pressure reactor equipped with heating and means of agitation. The reactor was sealed, flushed with $CO/H_2$ mixture and pressurized to 277 bars with a 1:1 molar $CO/H_2$ mixture. The mixture was heated to 220°C with rocking, the pressure allowed to rise to 450 bars and the reactor held at temperature for 18 hours.

On cooling the reactor pressure (105 bars) was noted and the excess gas removed. A two-phase liquid product (20.2 g) was recovered and samples were analyzed by glc and Karl Fischer titration. The bulk of the product material consisted of a grey-colored heavier phase (15 ml) which contained both acetic acid (6.1%) and propionic acid (1.1%) fractions.

Example 11

This Example illustrates the synthesis of acetic acid together with propionic acid and their esters directly from synthesis gas using as catalyst a ruthenium-containing compound in combination with a halide-free zirconium compound plus elemental iodine.

A mixture of ruthenium oxide, hydrate (4.0 mmoles), zirconium(IV) 2,4-pentanedionate (4.0 mmoles), and tetrabutylphosphonium bromide (10 g), and elemental iodine (4.0 mmoles) were charged to a glass liner, under $N_2$ purge, and transferred to the same 450 ml capacity pressure reactor as in Example 1. The reactor was sealed, flushed with $CO/H_2$ mixture, pressurized to 277 bars with 1:1 molar $CO/H_2$ mixture and heated to 220°C with rocking for 18 hours.

On cooling, the reactor pressure (94 bars) was noted and the excess gas removed. The product within the glass liner consisted of 29.9 g of a grey-green colored slurry.

Analysis of typical liquid samples showed the presence of:

15.1 wt.% acetic acid
4.4 wt.% propionic acid
1.0 wt.% methyl acetate
72.2 wt.% water

Example 12

A mixture of ruthenium(IV) oxide (4 mmoles) and cobalt iodide (8 mmoles) dispersed in tetrabutylphosphonium bromide (10.0 g) was transferred to a glass liner, under $N_2$ purge and charged to an 850 ml capacity pressure reactor equipped with heating and agitation means. The reactor was sealed, flushed with $CO/H_2$ and pressurized to 139 bars with 1:1 $CO/H_2$. The mixture is heated to 220°C with rocking, the pressure raised to 484 bars by $CO/H_2$ (1:1 molar) from a large surge tank, and the reactor held at temperature for 18 hr. Pressure in the reactor was maintained at about 487 bars by incremental addition of the $CO/H_2$ mixture from the surge tank. On cooling, the reactor pressure (287 bars) was noted, a typical gas sample taken and the excess gas removed. The emerald-green liquid product (16.4 g) showed no evidence of a solid fraction. Samples were analyzed by glc and Karl-Fischer titration and the following results were obtained:

63.8 wt.% acetic acid
12.1 wt.% propionic acid
10.1 wt.% ethyl acetate
7.1 wt.% ethyl propionate
3.5 wt.% propyl propionate.

Major product fractions are further identified by isolation and nmr, ir, etc.

Analysis of typical off-gas samples showed the presence of:

65.2% hydrogen
28.8% carbon monoxide
4.2% carbon dioxide
0.3% methane

Since the total catalyst charge to the glass liner was 13.3 g, the yield of liquid products was calculated to be:

$$\frac{16.4-13.8}{13.8}\times 100=24\%$$

Fractional distillation of a 5.8 g sample of the crude liquid product, under 0.5 mm Hg vacuum, produced a distillate sample comprising >80% acetic acid.

Examples 13 to 20

A number of additional Examples were completed using the same procedures as in Example 12 and utilizing a number of different catalyst and low-melting quaternary salts combinations. Data relating to these Examples are included in Table 2 which follows.

It may be noted that a number of combinations of ruthenium compounds with cobalt halide salts and with different initial cobalt-to-ruthenium atomic ratios, when dispersed in tetrabutylphosphonium halide salts, have been found to yield the desired carboxylic acids, e.g., acetic acid plus propionic acid.

TABLE 2[a]

| Example | Catalyst | Melt | Pres. bars. | Temp. (°C.) | Liquid product composition (wt.%)[b] HOAc | PrOOH | MeOAc | EtOAc+ MeOOPr | PrOAc+ EtOOPr | BuOOH+ PrOOPr | $H_2O$ | Liquid yield% |
|---|---|---|---|---|---|---|---|---|---|---|---|---|
| 12 | $RuO_2$—$2CoI_2$ | $Bu_4PBr$ | 484 | 220 | 63.8 | 12.1 | 0.5 | 10.1 | 7.1 | 3.5 | 0.5 | 24 |
| 13 | $RuO_2$—$CoI_2$ | $Bu_4PBr$ | 477 | 220 | 26.5 | 10.5 | 4.3 | 21.9 | 17.3 | 7.4 | 1.4 | 172 |
| 14 | $RuO_2$—$CoI_2$ | $Bu_4PBr$ | 477.5 | 220 | 24.6 | 5.4 | 4.6 | 22.8 | 14.5 | 5.1 | 5.2 | 83 |
| 15 | $RuO_2$—$\frac{1}{2}CoI_2$ | $Bu_4PBr$ | 484 | 220 | 28.1 | 0.4 | 6.4 | 30.7 | 16.1 | 5.1 | 1.1 | 196 |
| 16 | $Ru(acac)_3$—$CoI_2$ | $Bu_4PBr$ | 484 | 220 | 61.2 | 10.0 | 2.0 | 9.9 | 8.5 | 2.1 | 0.5 | 88 |
| 17 | $Ru_3(CO)_{12}$—$CoI_2$ | $Bu_4PBr$ | 484 | 220 | 48.5 | 24.1 | 0.9 | 9.8 | 7.4 | 3.9 | 0.6 | 88 |
| 18 | $RuO_2$—$CoBr_2$ | $Bu_4PBr$ | 465 | 220 | 22.3 | 0.9 | 2.0 | 18.3 | 8.2 | 2.2 | 11.4 | 105 |
| 19 | $RuO_2$—$CoCl_2$ | $Bu_4PBr$ | 484 | 220 | 3.4 | — | 13.0 | 34.0 | 15.2 | 4.1 | 2.5 | 392 |
| 20 | $RuO_2$—$CoCl_2$ | $Bu_4PCl$ | 484 | 220 | 5.1 | 2.3 | 21.8 | 37.3 | 4.8 | 2.2 | 3.7 | 321 |

[a]Charge: Ruthenium, 4.0 mmole; tetrabutylphosphonium salt, 10 g; Run Conditions: 1:1 molar ($CO/H_2$), 18 hrs.
[b]Designations: Acetic Acid (HOAC); Propionic Acid (PrOOH); Butyric Acid (BuOOH), Acetate Esters (MeOAc, EtOAc, PrOAc); Propionate Esters (MeOOPr, EtOOPr, PrOOPr).
[c]Variable pressure run, 277 bars $CO/H_2$ initial.

Example 21

This Example illustrates a further synthesis of acetic acid in high yield together with propionic acid and their esters directly from synthesis gas using a cobalt-ruthenium containing catalyst dispersed in tetrabutylphosphonium bromide.

A mixture of ruthenium(IV) oxide (4 mmoles) and cobalt(II) iodide (4 mmoles) dispersed in tetrabutylphosphonium bromide (10.0 g) was charged to a glass liner, under nitrogen purge, and transferred to a 450 ml capacity pressure reactor equipped with heating and means of agitation. The reactor was sealed, flushed with $CO/H_2$ mixture and pressurized to 277 bars with a 1:2 molar $CO/H_2$ mixture. The mixture was heated to 220°C with rocking, the pressure allowed to rise to 497.5 bars, and the reactor held at temperature for 18 hours.

On cooling, the reactor pressure (244.5 bars) was noted, a typical gas sample taken and the excess gas removed. A dark green liquid product (16.9 g) was recovered and samples were analyzed by glc and Karl-Fischer titration. The following results were obtained:

70.4 wt.% acetic acid
6.9 wt.% propionic acid
1.5 wt.% ethyl acetate
7.5 wt.% propyl acetate
0.5 wt.% water

Analysis of typical off-gas samples showed the presence of:

25% hydrogen
67% carbon monoxide
5.9% carbon dioxide
0.7% methane

Since the total catalyst charge to the glass liner was 12.0 g, the yield of liquid organics was calculated to be 41%.

Fractional distillation of a 12.1 g sample of the crude liquid product, under 0.05 mm Hg vacuum, produced a distillate fraction comprising >80% acetic acid.

Examples 22 to 25

Following the general procedure of Example 12, four additional Examples were completed utilizing different ruthenium-cobalt catalyst combinations in tetrabutylphosphonium bromide. Data relating to Examples 22—25 are included in Table 3 which follows.

It may be noted that:

a) for the ruthenium(IV) oxide—cobalt(II) iodide combination dispersed in tetrabutylphosphonium bromide, the addition of elemental iodine to the reaction mixture results in informal selectivity to acetic acid in the liquid product (see Examples 13, 22 and 23).

b) Acetic acid and propionic acid may be prepared directly from carbon monoxide and hydrogen using as a catalyst a ruthenium compound in combination with a halide-free cobalt compound plus a source of iodine. In these cases, acetic acid formation is illustrated for the ruthenium(IV) oxide—cobalt(III) acetylacetonate—iodine and ruthenium(IV) oxide—dicobalt octacarbonyl—iodine combinations (see Example 24 and 25).

TABLE 3[a]

| Example | Catalyst | Melt | Pres. bars. | Temp. (°C.) | Liquid product composition (wt.%)[b] HOAc | PrOOH | MeOAc | EtOAc+ MeOOPr | PrOAc+ EtOOPr | BuOOH+ PrOOPr | $H_2O$ | Liquid yield% |
|---|---|---|---|---|---|---|---|---|---|---|---|---|
| 13 | $RuO_2—CoI_2$ | $Bu_4PBr$ | 477 | 220 | 26.5 | 10.5 | 4.3 | 21.9 | 17.3 | 7.4 | 1.4 | 172 |
| 22 | $RuO_2—CoI_2—\frac{1}{2}I_2$ | $Bu_4PBr$ | 484 | 220 | 62.7 | 22.7 | 0.4 | 4.0 | 3.2 | 1.8 | 0.7 | 59 |
| 23 | $RuO_2—CoI_2—I_2$ | $Bu_4PBr$ | 484 | 220 | 86.3 | — | — | 6.3 | 2.1 | — | — | 59 |
| 24 | $RuO_2—Co(acac)_3—I_2$ | $Bu_4PBr$ | 484 | 220 | 36.6 | 12.0 | 2.9 | 18.8 | 11.5 | 4.7 | 1.1 | 105 |
| 25 | $RuO_2—\frac{1}{2}Co_2(CO)_8—I_2$ | $Bu_4PBr$ | 484 | 220 | 28.1 | 5.6 | 4.4 | 23.6 | 16.8 | 8.0 | 1.2 | 114 |

Charge: Ruthenium, 4.0 mmole; Tetrabutylphosphonium Bromide, 10 g; Run Conditions: 1:1 molar ($CO/H_2$); 18 hrs.
Designations as Per Table 2.

Comparative Example 26

In this Comparative Example, the catalyst utilized consisted only of the halide-containing cobalt compound dispersed in the low-melting quaternary salt. There was no ruthenium catalyst component in this Example. No acetic or propionic acids were formed in the absence of the ruthenium component.

Cobalt iodide (8 mmoles) dispersed in tetrabutylphosphonium bromide (10.0 g) was transferred to a glass liner, under $N_2$ purge and charged to an 850 ml capacity pressure reactor equipped with heating and agitation means. The reactor was sealed, flushed with $CO/H_2$ and pressurized to 139 bars with 1:1 molar $CO/H_2$. The mixture was heated to 220°C with rocking, the pressure raised to 483 bars by $CO/H_2$ (1:1 molar) from a large surge tank, and the reactor held at temperature for 18 hr. Pressure in the reactor was maintained at about 487 bars by incremental addition of the $CO/H_2$ mixture from the surge tank. On cooling, the reactor pressure was released; the product within the glass liner consisted of 12.5 g of deep-blue liquid material. Samples of this liquid which were analyzed by glc showed no evidence for the presence of acetic or propionic acids. The yield of liquid product was calculated to be <2%.

Example 27

A mixture of ruthenium(III) acetylacetonate (2 mmoles) and cobalt iodide (2 mmoles) dispersed in tetrabutylphosphonium bromide (10.0 g) was transferred to a glass liner, under $N_2$ purge and charged to an 850 ml capacity pressure reactor equipped with heating and agitation means. The reactor was sealed, flushed with $CO/H_2$ and pressurized to 139 bars with 1:1 molar $CO/H_2$. The mixture was heated to 220°C with rocking, the pressure raised to 484 bars by $CO/H_2$ (1:1 molar) from a large surge tank, and the reactor held at temperature for 18 hr. Pressure in the reactor was maintained at about 480 bars by incremental addition of the $CO/H_2$ mixture from the surge tank. On cooling, the reactor pressure (277 bars) was noted, a typical gas sample taken and the excess gas removed. The emerald-green liquid product (20.6 g) showed no evidence of a solid fraction. Samples were analyzed by glc and Karl-Fischer titration. The following results were obtained:

59.8 wt.% acetic acid
11.7 wt.% propionic acid
9.6 wt.% ethyl acetate
7.5 wt.% ethyl propionate
1.5 wt.% propyl propionate

Major product fractions were further identified by isolation and nmr, ir, etc. The liquid yield was estimated to be 81%.

The crude product liquid after analysis was subject to distillation, *in vacuo*, and the acetic acid and propionic acid, plus their esters, were isolated as distillate fractions. The residual ruthenium-cobalt catalyst (8.9 g) was returned to the glass lined reactor, the reactor sealed, flushed with $CO/H_2$, and pressurized to 139 bars with 1:1 molar $CO/H_2$. The mixture was heated to 220°C with rocking, the pressure raised to 484 bars by $CO/H_2$ (1:1 molar) from a large surge tank, and the reactor held at temperature for 18 hr. In this manner the synthesis of acetic plus propionic acids is repeated, and the latter are again recovered from the iodide liquid product (15.2 g) by fractional distillation in vacuo. The CO-hydrogenation to acetic and propionic acids was repeated using the same ruthenium-cobalt catalyst sample an additional two times. After each cycle, the crude liquid products were subject to analysis. The results for the four catalyst cycle experiment are summarized in Table 4 below.

TABLE 4[a]

| Ruthenium-cobalt catalyst cycle | Liquid product composition (wt.%)[b] HOAc | PrOOH | EtOAc MeOOPr | PrOAc EtOOPr | BuOOH PrOOPr | Liquid yield (%) |
|---|---|---|---|---|---|---|
| I | 59.8 | 11.7 | 9.6 | 7.5 | 1.5 | 81 |
| II | 65.8 | 5.5 | 9.5 | 2.0 | —— | 88 |
| III | 61.5 | 10.1 | 10.6 | 8.3 | 1.8 | 90 |
| IV | 56.0 | 4.6 | 18.3 | 10.5 | 2.0 | 33 |

[a]Charge: $Ru(acac)_3$, 2.0 mmole; $CoI_2$, 2.0 mmole; $Bu_4PBr$, 10 g; Run Conditions: 484 bars; 220°C; $CO/H_2$ (1:1 molar), 18 hr.
[b]Designations as Per Table 2.

**Claims**

1. A process for reacting mixtures of carbon monoxide and hydrogen at a temperature of at least 150°C

and a pressure of at least 35 bars in the presence of a catalyst comprising a ruthenium compound and a co-catalyst dispersed in a low melting quaternary ammonium or phosphonium base or salt, characterized in that the co-catalyst is a halogen-containing cobalt, titanium or zirconium compound or a halogen-containing or halogen-free cobalt, titanium or zirconium compound being employed with iodine or an iodine-containing compound and said mixtures of carbon monoxide and hydrogen do not include other gases that are reactive under co-hydrogenation conditions, whereby said carbon monoxide and hydrogen are converted selectively into acetic acid, propionic acid and esters thereof.

2. A process according to Claim 1 characterized in that the cobalt, titanium or zirconium co-catalyst is a halogen-containing compound and iodine or an iodine compound is additionally incorporated in the catalyst.

3. A process according to Claim 1 or 2 characterized in that the cobalt, titanium or zirconium co-catalyst is cobalt(II) chloride, bromide or iodide, bis(cyclopentadienyl) titanium dichloride, bis(cyclopentadienyl) titanium hydrochloride, titanium(IV) chloride or bromide, bis(cyclopentadienyl) zirconium dichloride, bis(cyclopentadienyl) zirconium hydrochloride, zirconium(IV) chloride or bromide, or zirconium dichloride oxide.

4. A process according to Claim 1 characterized in that the cobalt titanium or zirconium co-catalyst is a halogen-free compound employed in association with iodine or an iodine compound.

5. A process according to Claim 4 characterized in that the halogen-free co-catalyst comprises cobalt(III) acetylacetonate, dicobalt octacarbonyl, titanium(IV), 2,4-pentanedionate, titanium(IV) methoxide, titanium oxide, zirconium(IV) 2,4-pentanedionate, zirconium(IV) methoxide or zirconium diacetate oxide.

6. A process according to any preceding Claim characterized in that the temperature is from 150 to 350°C.

7. A process according to any preceding Claim characterized in that the pressure is from 135 to 700 bars.

8. The process according to any preceding Claim characterized in that the ruthenium-containing compound is an oxide of ruthenium, a ruthenium salt of a mineral acid, a ruthenium salt of an organic carboxylic acid, or a ruthenium carbonyl or hydrocarbonyl derivative.

**Patentansprüche**

1. Verfahren zur Umsetzung von Gemischen aus Kohlenmonooxid und Wasserstoff bei einer Temperatur von mindestens 150°C und einem Druck von mindestens 35 bar in Gegenwart eines Katalysators, der eine Ruthenium-Verbindung und einen Co-Katalysator enthält oder daraus besteht, wobei der Katalysator dispergiert in einer niedrigschmelzenden quarternären Ammonium- oder Phosphonium-Base oder -Salz vorliegt, dadurch gekennzeichnet, daß der Co-Katalysator eine Halogen enthaltende Cobalt-, Titan- oder Zirkon-Verbindung oder eine halogenhaltige oder halogenfreie Cobalt-, Titan- oder Zirkon-Verbindung ist, welche zusammen mit Jod oder eine Jodid enthaltenden Verbindung eingesetzt wird, und diese Gemische von Kohlenmonoxid und Wasserstoff keine weiteren Gase enthalten, die unter Co-Hydrierbedingungen reaktiv sind, wobei das Kohlenmonoxid und Wasserstoff selektiv in Essigsäure, Propionsäure und deren Ester umgesetzt werden.

2. Ein Verfahren nach Anspruch 1, dadurch gekennzeichnet, daß der Cobalt-, Titan- oder Zirkon-Co-Katalysator Halogen enthaltende Verbindung ist und Jod oder eine Jodid-Verbindung zusätzlich in den Katalysator einverleibt wird.

3. Ein Verfahren nach Anspruch 1 oder 2, dadurch gekennzeichnet, daß der Cobalt-, Titan- oder Zirkon-Co-Katalysator Cobalt(II)chlorid, -bromid oder -jodid, Bis(cyclopentadienyl)-titandichlorid, Bis(cyclopentadienyl)-titanhydrochlorid, Titan(IV)chlorid oder -bromid, Bis(cyclopentadienyl)-zirkondichlorid, Bis-(cyclopentadienyl)-zirkonhydrochlorid, Zirkon(IV)chlorid oder -bromid, oder Zirkondichloridoxid ist.

4. Ein Verfahren nach Anspruch 1, dadurch gekennzeichnet, daß der Cobalt-, Titan- oder Zirkon-Co-Katalysator eine halogenfreie Verbindung ist, die zusammen mit Jod oder einer Jodid-Verbindung eingesetzt wird.

5. Ein Verfahren nach Anspruch 4, dadurch gekennzeichnet, daß der halogenfreie Cokatalysator Cobalt(III)acetylacetonat, Dicobaltoctacarbonyl, Titan(IV)2,4-pentandionat, Titan(IV)methoxid, Titanoxid, Zirkon(IV)2,4-pentandionat, Zirkon(IV)methoxid oder Zirkondiacetatoxid enthält oder daraus besteht.

6. Ein Verfahren nach einem der vorhergehenden Ansprüche, dadurch gekennzeichnet, daß die Temperatur 150 bis 350°C beträgt.

7. Ein Verfahren nach einem der vorhergehenden Ansprüche, dadurch gekennzeichnet, daß der Druck 135 bis 700 bar beträgt.

8. Das Verfahren nach einem der vorhergehenden Ansprüche, dadurch gekennzeichnet, daß die Ruthenium enthaltende Verbindung ein Oxid des Rutheniums, ein Ruthenium-Salz einer Mineralsäure, ein Ruthenium-Salz einer organischen Carbonsäure oder ein Rutheniumcarbonyl oder -hydrocarbonyl-Derivat ist.

**Revendications**

1. Procédé pour faire réagir des mélanges d'oxyde de carbone et d'hydrogène à une température d'au

moins 150°C et sous une pression d'au moins 35 bars en présence d'un catalyseur comprenant un composé du ruthénium et un co-catalyseur dispersé dans une base de sel d'ammonium ou de phosphonium quaternaire à bas point de fusion, caractérisé en ce que le co-catalyseur est un composé halogéné du cobalt, du titane ou du cobalt ou un composé halogéné ou un composé du cobalt du titane ou du zirconium exempt d'halogène utilisé avec de l'iode ou un composé contenant de l'iode, et en ce que ces mélanges d'oxydes de carbone et d'hydrogène ne contiennent pas d'autres gaz qui soient réactifs dans les conditions de co-hydrogénation, grâce à quoi, cet oxyde de carbone et cet hydrogène sont transformés sélectivement en acide acétique, acide propionique et leurs esters.

2. Procédé suivant la revendication 1, caractérisé en ce que le co-catalyseur de cobalt, de titane ou de zirconium est un composé halogéné et en ce que de l'iode ou un composé de l'iode est incorporé en outre au catalyseur.

3. Procédé suivant les revendications 1 ou 2, caractérisé en ce que le co-catalyseur de cobalt, de titane ou de zirconium est du chlorure, du bromure ou de l'iodure de cobalt (II), du dichlorure de bis(cyclopentadiényle) titane, du chlorhydate de bis(cyclopentadiényle)titane, du chlorure ou du bromure de titane (IV), du dichlorure de bis(cyclopentadiényle) zirconium, du chlorhydrate de bis(cyclopentadiényle) zirconium, du chlorure ou du bromure de zirconium (IV), ou du dichlorure oxyde de zirconium.

4. Procédé suivant la revendication 1, caractérisé en ce que le co-catalyseur de cobalt, de titane, ou de zirconium est un composé exempt d'halogènes utilisé en association avec de l'iode ou un composé de l'iode.

5. Procédé suivant la revendication 4, caractérisé en ce que le co-catalyseur exempt d'halogène comprend de l'acétyle acétonate de cobalt (III), du dicobalt octacarbonyle, du 2,4-pentandionate de titane (IV), du méthylate de titane (IV), de l'oxyde de titane, du 2,4-pentadionate de zirconium (IV), du méthylate de zirconium (IV) ou du diacétate oxyde de zirconium.

6. Procédé suivant l'une quelconque des revendications précédentes, caractérisé en ce que la température est de 150 à 350°C.

7. Procédé suivant l'une quelconque des revendications précédentes, caractérisé en ce que la pression est de 135 à 700 bars.

8. Procédé suivant l'une quelconque des revendications précédentes, caractérisé en ce que le composé contenant du ruthénium est un oxyde de ruthénium, un sel de ruthénium d'un acide minéral, un sel de ruthénium d'un acide carboxylique organique ou un dérivé carbonylé ou hydrocarbonylé du ruthénium.